# EUROPÄISCHE PATENTSCHRIFT

(11) **EP 0 442 843 B1**
(45) Veröffentlichungstag und Bekanntmachung des Hinweises auf die Patenterteilung: **09.09.1998**
(21) Anmeldenummer: 91810073.6
(22) Anmeldetag: 01.02.1991
(51) Int. Cl.: G01N 33/86, A61K 35/62, A61L 33/00, C12Q 1/56

(54) **Inhibitoren zur antikoagulierenden Vorbehandung von Blutproben**
Inhibitors for the anticoagulant pretreatment of blood samples
Inhibiteur pour le prétraitement anticoagulant des échantillons sanguins

(30) Priorität: 14.02.1990 CH 478/90
(43) Veröffentlichungstag der Anmeldung: 21.08.1991
(73) Patentinhaber: Pentapharm A.G., CH-4052 Basel (CH)
(72) Erfinder: Stocker, Kurt F., CH-4147 Aesch (CH); Wenzel, Ernst, A-1010 Wien (CH)
(74) Vertreter: Braun, André, jr.

(56) Entgegenhaltungen:
- WO-A-79/00638
- DIE PHARMAZIE, Band 43, Nr. 11, November 1988, Seiten 737-744, VEB Verlag Volk und Gesundheit, Berlin, DE; P. WALSMANN: "Über den Einsatz des spezifischen Thrombininhibitors Hirudin für diagnostische und biochemische Untersuchungen"
- DE PHARMAZIE, Band 41, Nr. 1, Juli 1986, Seite 509, VEB Verlag Volk und Gesundheit, Berlin, DE; H. HORN et al.: "Synthese von Nalpha-(2- Naphthylsulfonylglycyl)-4-aminomethyl- und -4-amino-phenylalaninpiperidid als
- Thrombininhibitoren"

## Beschreibung

In der Blutflüssigkeit (Plasma) suspendierte Zellen und Korpuskeln üben verschiedene lebenswichtige Funktionen aus. So erfüllen weisse Blutkörperchen (Leukozyten) vielfältige Aufgaben in der Infektionsabwehr. Rote Blutkörperchen (Erythrozyten) wirken als Vehikel für den Transport von Sauerstoff und Kohlendioxid, und Blutplättchen (Thrombozyten) dienen der Blutstillung und der Reparatur verletzter Blutgefässe. Die Feststellung von Normabweichungen in Anzahl, Aspekt, Eigenschaften und Funktionen dieser Zellen und Korpuskeln ermöglicht Rückschlüsse auf bestehende Erkrankungen oder vererbte Anomalien. Der Bestimmung der Funktionen und Zustandsänderungen der Blutzellen und Blutkorpuskel (im folgenden durch die Abkürzung "BBFZ-Bestimmungen" bezeichnet) kommt daher eine beträchtliche diagnostische Aussagekraft zu. Häufig angewandte BBFZ-Bestimmungen sind die Bestimmungen der Blutsenkungsgeschwindigkeit oder Erythrozytensedimentationsrate, der Erythrozytendeformierbarkeit, der Plättchenaggregation, der Plättchenadhäsivität und der Freisetzung biologisch aktiver Substanzen aus Blutplättchen. Dazu gehören auch verschiedene cytochemische Tests zur Bestimmung von Leukozytenfunktionen, wie zum Beispiel die Peroxidasereaktion oder der Nitroblautetrazoliumtest.

Derartige BBFZ-Bestimmungen sind jedoch durch die spontane Gerinnungsfähigkeit des Blutes erschwert. Im Plasma in einem labilen Ruhezustand vorliegende Blutgerinnungsfaktoren werden nämlich durch Kontakt von austretendem Blut mit einer fremden Oberfläche aktiviert und gehen eine Wechselwirkung ein, die zunächst zur Bildung der Proteinase Thrombin aus einer inaktiven Vorstufe führt. Thrombin bewirkt schliesslich die Umwandlung des kolloidal gelösten Plasmaproteins Fibrinogen in das gelierte Fibrin; das Blut geht dadurch vom flüssigen in den geronnenen, gelierten Zustand über. Zellen und Korpuskeln werden dabei in das Fibringerinnsel eingeschlossen und können nicht mehr einer BBFZ-Bestimmung unterzogen werden. Zur Durchführung von BBFZ-Bestimmungen in Blut muss daher dessen Gerinnungsfähigkeit durch geeignete Massnahmen unterbunden werden.

Da mehrere an der Blutgerinnung beteiligte Reaktionen nur in Anwesenheit von Calciumionen ablaufen, kann die Gerinnungsfähigkeit des Blutes durch Entzug von Calciumionen mittels Ionenaustauschern, Fällungsreagenzien oder Komplexbildnern vollkommen aufgehoben werden. Häufig bei der Entnahme von Blutproben verwendete calciumbindende, antikoagulierende Zusätze sind Natriumcitrat, Natrium-, Kalium-oder Ammoniumoxalat, Natriumsulfat, Salze der Ethylendiamintetraessigsäure sowie Gemische dieser Stoffe. Calciumionen-bindende oder fällende Zusätze eignen sich jedoch nur dann, wenn die betreffende Blutprobe zu einer calcium-unabhängigen BBFZ-Bestimmung verwendet werden soll. BBFZ-Bestimmungen von calciumabhängigen Funktionen, wie z.B. Adhäsions-, Aggregations- und Freisetzungreaktionen von Blutplättchen, oder Untersuchungen intracellulärer oder plasmatischer Elektrolytkonzentrationen können nur mit Antikoagulantien durchgeführt werden, welche die Elektrolyt-und im Besonderen die Calciumionenkonzentration nicht verändern. Ausserdem hängen verschiedene Zell- und Korpuskelfunktionen in Blut und Plasma nicht nur von Calciumionen, sondern auch von anderen 2-wertigen Kationen, wie z.B. Magnesium-, Zink- und Kupferionen, ab, welche durch Salze der Ethylendiamintetraessigsäure oder andere als Blutantikoagulantien verwendete Komplexbildner gebunden werden. Magnesiumionen werden überdies durch Oxalatsalze als schwerlösliches Magnesiumoxalat ausgefällt.

Da Natriumoxalat oder Natriumcitrat eine spontane Plättchenaggregation nicht zu verhindern vermag, wird zur Gewinnung von plättchenreichem Plasma meistens eine saure, glucosehaltige Natriumcitratlösung als Antikoagulans bei der Entnahme der Blutproben eingesetzt. Saure Citratlösungen wirken aber nicht nur stabilisierend auf Blutplättchen, sondern hemmen ganz allgemein ihre Funktionen und reduzieren ihre Reaktionsfähigkeit auf chemische und physikalische Stimuli.

Um die rasche Durchmischung von Antikoagulans und Blut zu erreichen, wird üblicherweise ein graduierter Behälter mit dem erforderlichen Volumen einer Antikoagulanslösung beschickt und hierauf mit frisch aus einer Vene entnommenem Blut bis zur entsprechenden Eichmarke aufgefüllt. Bei diesem Vorgehen können allerdings die betreffenden BBFZ-Bestimmungen ausschliesslich in verdünntem und nicht in nativem Blut durchgeführt werden. Um eine Beeinflussung der Bestimmungen durch unterschiedliche Verdünnungseffekte auszuschliessen, muss unbedingt auf eine strikte Einhaltung des richtigen Mischungsverhältnisses zwischen Blut und Antikoagulans geachtet werden. Eine gleichbleibende Probenverdünnung ist jedoch dann nicht zu erreichen, wenn für die zwar in genormter Menge vorgelegte Antikoagulanslösung die erforderliche Blutmenge nicht beigebracht werden kann, weil sich die Blutentnahme schwierig gestaltet. Dies ist vor allem bei Blutentnahmen bei Kleinkindern häufig der Fall. Die verwendeten Antikoagulantien müssen ferner als isotonische Lösungen vorgelegt werden, weil osmotische Veränderungen des Milieus die Eigenschaften und Funktionen von Blutzellen und Blutkorpuskeln stark beeinflussen können. Im Extremfall kann beispielsweise durch Hypotonie des Mediums ein Platzen der Erythrozyten, eine sogenannte Haemolyse, verursacht werden. Die calciumbindenden Antikoagulanslösungen müssen ausserdem so beschaffen sein, dass der physiologische pH-Wert erhalten bleibt, denn insbesondere Plättchenfunktionen werden durch eine Senkung des pH-Wertes sehr stark beeinflusst.

Bei der Bestimmung der Blutsenkungsgeschwindigkeit (BSG) misst man in einer Blutprobe das Ausmass der in einer gegebenen Zeitspanne erfolgenden, auf einem Dichteunterschied zwischen Blutflüssigkeit und Zellen beruhenden Sedimentation der roten Blutkörperchen. Eine erhöhte BSG zeigt das Vorliegen eines aktiven Krankheitsprozesses, wie zum Beispiel einer Infektion, einer Gefässerkrankung oder eines Krebsleidens an. Eine verminderte BSG wird beispielsweise bei abnorm geformten Erythrozyten (Sichelzellanämie), einer pathologisch erhöhten Erythrozytenzahl (Polycythaemie) oder einem erniedrigten Fibrinogenspiegel (Hypofibrinogenämie) beobachtet.

Die BSG hängt von der Neigung zur Aggregation und Bildung rollenförmiger Verklumpungen der Erythrozyten ab. Diese Rollenbildung wird durch Eigenschaften der Erythrozyten selbst, durch Scherkräfte, Temperatur, Viskosität des Blutplasmas sowie durch die Wirkung brückenbildender Macromoleküle bestimmt. Unter normalen Verhältnissen wirkt die elektronegative Ladung der Erythrozyten einer Aggregation entgegen und die normale BSG entspricht der Sedimentation freier, nicht aggregierter Erythrozyten. Rollenförmige Aggregation kommt zustande, wenn sich bestimmte Plasmaproteine, wie z.B. Fibrinogen, α- und β-Globuline, an der Zelloberfläche anreichern und die elektronegative, sich gegenseitig abstossende Ladung der Erythrozyten abschwächen. Rollenförmige Aggregate besitzen, bezogen auf ihre Oberfläche, ein grösseres Partikelgewicht als freie Erythrozyten und sedimentieren darum rascher.

Die Entstehung von löslichen Fibrinkomplexen im Blut, ausgelöst durch Thrombin oder thrombinartige Enzyme, verursacht eine drastisch verminderte BSG (Blättler et al., Thromb. Res. 4, 787, 1974). Eine ausführliche Beschreibung der BSG-Bestimmung findet sich bei K.-G. v. Boroviczeny et al., Qualitätssicherung im Medizinischen Laboratorium, Springer-Verlag, Instand Schriftenreihe, Band 5, 553-572, (1987). Alle beschriebenen Methoden haben den Nachteil, dass sie zur Gewinnung der Blutprobe Lösungen von Antikoagulantien erfordern, welche die Plasmaviskosität sowie die Wirkung brückenbildender Macromoleküle bereits durch den Verdünnungseffekt beeinflussen und das Ergebnis der BSG-Messung verändern (siehe H. Kiesewetter und H. Radtke: Die Blutsenkung: Ein altes klinisches Verfahren unter neuen Aspekten, Klin. Wochenschr. 61, 621-624, 1983). Eine Variation des Mischungsverhältnisses von Blut und Antikoagulans um 5% bewirkt aber bereits eine Abweichung von 10% des gemessenen Stundenwertes.

Eine bedeutende Fehlerquelle bei BBFZ-Bestimmungen in antikoagulierten Blutproben liegt ferner in einer möglichen mikrobiellen Kontamination der Antikoagulanslösung. Eine mikrobielle Kontamination der Antikoagulanslösung kann dadurch ausgeschaltet werden, dass man Röhrchen oder Spritzen, die der Blutentnahme dienen sollen, im voraus mit Antikoagulanslösung beschickt, diese Behältnisse keimundurchlässig verpackt und sterilisiert. Derart speziell für die BSG-Bestimmung vorbereitete Blutentnahmegeräte sind im Fachhandel erhältlich und erleichtern die Durchführung der Bestimmungen zwar beträchtlich, bergen aber nach wie vor die Gefahr eines falschen Mischungsverhältnisses von Blut und Antikoagulans. Ferner können mit diesen Blutaufnahmegeräten nur calciumunabhängige BBFZ-Bestimmungen und zwar nur in verdünnten Blutproben durchgeführt werden.

Um den Verdünnungseffekt durch die vorgelegte Antikoagulanslösung bei der BSG-Bestimmung zu eliminieren, wurde versucht, die Methode so zu modifizieren, dass man die als Antikoagulans dienende Oxalatlösung in Blutentnahmeröhrchen abfüllte und bei 60° trocknete, um so eine Verdünnung der Blutprobe auszuschliessen. Zur Antikoagulation von 1 ml Blut werden jedoch ca. 20 mg Oxalatsalze benötigt, welche nach dem Trocknungsvorgang als relativ grobe Partikel vorliegen, die in der Blutprobe nur langsam und nur nach entsprechendem Schütteln in Lösung gehen. Beim Schütteln muss aber das Entstehen von Luftblasen unbedingt vermieden werden, da diese die Erythrozytensedimentation stören und so das Bestimmungsergebnis verfälschen. Die Auswirkungen dieser Fehlerquelle hängen jeweils von der individuellen Arbeitsweise ab und lassen sich bei der Verwendung von getrocknetem Oxalat weder ganz ausschliessen noch standardisieren.

Bei Verwendung von Oxalat- oder Citratsalzen als Antikoagulantien entstehen ausserdem unlösliche Calciumsalze, welche an Blutzellen und -korpuskeln adhärieren, deren spezifisches Gewicht und ihre Oberflächenbeschaffenheit verändern und dadurch die verschiedenen BBFZ-Bestimmungen beeinflussen können. Ausserdem können Calciumcitrat und Calciumoxalat Plasmaproteine adsorbieren und auf diese Weise die BSG- und andere BBFZ-Bestimmungen entscheidend beeinflussen.

Es wurde versucht, den Einsatz von flüssigen, calciumionen eliminierenden Antikoagulantien dadurch zu umgehen, dass man die zur Blutentnahme verwendeten Behältnisse mit dem bereits in geringer Konzentration wirksamen Gerinnungshemmer Heparin beschichtet und trocknet. Heparin ist ein Mucopolysaccharid mit einem Molekulargewicht von ca. 17'000 Dalton, welches mit dem im Blutplasma enthaltenen Antithrombin III, einem Protein mit einem Molekulargewicht von 61'000 Dalton, einen hochmolekularen Inhibitorkomplex bildet, welcher α-Thrombin, das Schlüsselenzym der Blutgerinnung, sowie die Gerinnungsfaktoren IXa, Xa, XIa und XIIa hemmt und dadurch zwar die Gerinnung, nicht aber eine spontane Viskositätsveränderung der Blutprobe verhindert. Heparin hat sich darum als ungeeignet zur Durchführung einer BSG-Bestimmung nach Wintrobe erwiesen (S.S. Raphael (Ed) Lynch's Medical Laboratory Technology, Vol. II, 3rd edition, W.B. Saunders Co., Philadelphia, 1976). Heparin bindet den bei der Thrombozytenaggregation freigesetzten Plättchenfaktor 4 (PF 4) und wird dadurch selbst inaktiviert. Wegen dieser Wechselwirkung kann Heparin nicht als Blutantikoagulans bei einer Bestimmung der PF 4-Freisetzung angewendet werden. Heparin übt ausserdem eine starke plättchenaggregierende Wirkung aus und kann darum auch nicht als Antikoagulans zur Durchführung von Plättchenfunktionsbestimmungen verwendet werden. Wegen dieser aggregierenden Wirkung, die ausserdem je nach Ursprung und Reinheitsgrad des Heparins verschieden sein kann, eignet sich Heparin sogar nicht einmal als Antikoagulans zur mikroskopischen oder automatischen Auszählung der Plättchenmenge in Plasma. Heparin und Calciumkomplexbildner, wie zum Beispiel Ethylendiamintetraessigsäure, setzen die Plättchenadhäsivität herab und eignen sich darum nicht als Antikoagulantien für Blutproben zur Bestimmung der Plättchenadhäsivität. Bei cytochemischen Farbreaktionen an Leukozyten kann das elektronegativ geladene Heparin zu unerwünschter Bindung von Farbreagenzien führen.

Das zur Entfaltung der gerinnungshemmenden Wirkung von Heparin unerlässliche im Plasma von gesunden Probanden in ausreichender Menge vorliegende Antithrombin III ist bei verschiedenen Erkrankungen, wie zum Beispiel Lebercirrhose, Hepatitis, Venenthrombose, disseminierte intravasale Gerinnung, Septicaemie, oder unter verschiedenen Medikationen, wie zum Beispiel bei Einnahme oraler Empfängnisverhütungsmittel, oder beim Vorliegen eines vererbten Antithrombin III-Mangels stark vermindert. Bei den genannten Zuständen kann darum die Antikoagulanswirkung von Heparin bei der Gewinnung von Blutproben für analytische Zwecke ganz oder teilweise versagen.

Überraschenderweise wurde nun gefunden, dass sich der Thrombininhibitor Hirudin aus dem Blutegel Hirudo medicinalis, im Gegensatz zu Heparin, hervorragend als Antikoagulans zur antikoagulierenden Vorbehandlung von Blutproben für BBFZ-Bestimmungen eignet. Hirudin verhindert bereits in sehr geringer Konzentration sowohl die Gerinnung als auch eine spontane Viskositätsänderung von Blutproben, ist sehr gut wasserlöslich, und ein mehrfacher Überschuss über die effektiv erforderliche Menge beeinflusst die Zell- und Korpuskelfunktionen nicht. Hirudin übt überdies auf Plättchen keine aggregierende Wirkung aus und hat keinerlei Einfluss auf die thrombinunabhängigen Funktionen und den Zustand der Blutzellen und Blutkorpuskeln. Darum erweist sich Hirudin auch bei der Bestimmung der Thrombozytenadhäsivität, der Leukozytenadhäsivität und der Thrombozytensedimentation als wirkungsvolles, mit den empfindlichen Testsystemen kompatibles Antikoagulans, welches dem Blut entweder in Form einer hochkonzentrierten Lösung oder in trockener Form beigemengt werden kann. Hirudin eignet sich darum besonders gut zur trockenen Beschichtung von Blutentnahme- und Testgeräten, weil es sich rasch in der Blutflüssigkeit auflöst und so hoch im Überschuss dosiert werden kann, dass sogar unvollständige Auflösung oder mangelhafte Durchmischung von Antikoagulans und Blutprobe sowie Über- oder Untervolumen der entnommenen Probemenge das Bestimmungsergebnis nicht beeinflussen. Dank seiner hohen spezifischen Inhibitoraktivität eignet sich Hirudin ganz besonders auch zum Anbringen einer löslichen Antikoagulans-Schicht in Microsedimentationsröhrchen, welche zur Bestimmung der BSG in Kapillarblut eingesetzt werden und welche wegen ihres kleinen Lumens nur eine sehr beschränkte Aufnahmefläche für das Antikoagulans bieten. Im Gegensatz zu Heparin, welches sowohl die Aggregation von Plättchen auslösen als auch die durch stimulierende Agenzien hervorgerufene Plättchenaggregation, je nach Agens, hemmen oder auch potenzieren kann, beeinflusst Hirudin thrombinunabhängige Thrombozytenfunktionen nicht. Im Gegensatz zu Heparin bindet Hirudin den Plättchenfaktor 4 nicht. Hirudin wird somit durch dieses aus Plättchen während ihrer Aggregation und viskösen Metamorphose freigesetzte Protein nicht inaktiviert. Im Gegensatz zu Heparin, das seine antikoagulierende Wirkung nur bei ausreichender Konzentration des im Plasma enthaltenen Cofaktors, Antithrombin III, entfalten kann, ist Hirudin ein von Plasma-Cofaktoren vollständig unabhängiger Inhibitor, der seine Wirkung auch im pathologisch veränderten Blut von Patienten mit verschiedensten Erkrankungen entfalten kann. Als Polypeptid bindet Hirudin auch keine für Leukozytenfunktionsbestimmungen eingesetzte basische Farbstoffe, welche mit dem stark sauren Polysaccharid Heparin unlösliche Komplexe bilden. Schliesslich bindet Hirudin auch keine zweiwertige Kationen, insbesondere Calcium-, Magnesium-, Zink- und Kupferionen. Infolgedessen werden von diesen Ionen abhängige Zell- und Korpuskelfunktionen durch Hirudin nicht gestört.

Im Vergleich zum hochmolekularen Heparin-Antithrombin III-Komplex ist Hirudin mit einem Molekulargewicht von lediglich ca. 7'000 Dalton ein kleines Polypeptid, welches in mehreren, als Inhibitor wirksamen Isoformen von der Speicheldrüse des Blutegels sezerniert wird. Bis heute wurden elf verschiedene molekulare Hirudinvarianten aus Speicheldrüsen von Blutegeln isoliert und charakterisiert. Von der mengenmässig dominierenden Variante HV-1 wurde die vollständige Aminosäuresequenz ermittelt. Als eine strukturelle Besonderheit von HV-1 wurde ein sulfatierter Tyrosinrest in Position 63 ermittelt. Für die übrigen natürlichen Hirudinvarianten wurden bis anhin Teile der Struktur aufgeklärt, und es konnte das natürliche Vorkommen von sowohl sulfatierten als auch nichtsulfatierten Hirudinvarianten nachgewiesen werden. Eine ausführliche Beschreibung natürlicher und gentechnologisch gewonnener, rekombinanter Hirudine findet sich bei F. Markwardt, Development of hirudin as an antithrombotic agent, Seminars in Thrombosis and Hemostasis 15, 269-282 (1989). Hirudin hemmt α-Thrombin und als niedermolekularer, beweglicher Inhibitor auch dessen enzymatisch aktive Vorstufe Meizothrombin, welches bekanntlich durch den hochmolekularen Heparin-Antithrombin III-Komplex nicht gehemmt wird. Eine chemische oder enzymatische Desulfatierung von Hirudin beeinträchtigt seine Inhibitorwirkung nicht, so dass auch rekombinantes Desulfatohirudin ein vollwirksamer Inhibitor von Thrombin ist.

Es wurde ferner gefunden, dass sich Hirudin, welches nicht aus Blutegeln, sondern aus Microorganismen, deren Genom mit dem für Hirudin codierenden Gen rekombiniert wurde, gleich gut wie natürliches Hirudin zur antikoagulierenden Vorbehandlung von Blutproben für BBFZ-Bestimmungen eignet. Schliesslich wurde gefunden, dass sich auch niedermolekulare, synthetische Thrombin- und Meizothrombininhibitoren, wie zum Beispiel Na-(2-naphthylsulfonylglycyl)-D,L-amidinophenylalaninpiperidid (abgekürzt NAPAP), für den gleichen Zweck eignen.

"Pharmazie, Jahrgang 41, S. 509, 1986" erwähnt daß N-alpha-(2-Naphthylsulfonylglycyl)-4-aminomethyl und -4-amino-phenylalaninpiperidid als Thrombininhibitoren wirksam sind.

Aus "Pharmazie, Jahrgang 43, S. 737-744, 1988, ist die Meizothrombin- und Thrombin-hemmende Wirkung von Hirudin bekannt.

Die vorliegende Erfindung betrifft nun ein Verfahren zur Bestimmung von meizothrombin- und thrombinunabhängigen Funktionen und Zustandsänderungen von Blutzellen und Blutkorpuskeln, das durch die Merkmale in Patentanspruch 1 gekennzeichnet ist.

Als Inhibitor wird Hirudin oder Desulfatohirudin oder eine Verbindung der Formel 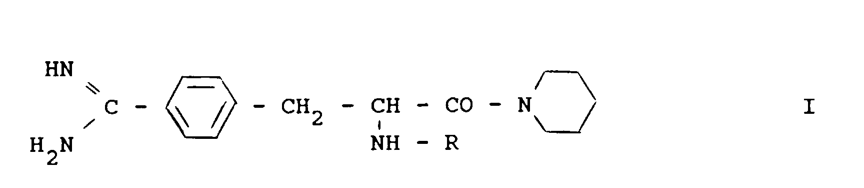 in welcher R eine Toluolsulfonylglycyl-, α-Naphthylsulfonylglycyl oder β-Naphthylsulfonylglycyl-Gruppe darstellt, oder ein Gemisch von Hirudin oder Desulfatohirudin mit einer Verbindung der Formel I, verwendet.

Die an sich bekannten Verbindungen der Formel I weisen die gleichen Eigenschaften und die gleichen Vorteile gegenüber Heparin auf wie Hirudin.

Als Hirudinspecies kann man das aus dem Blutegel H. medicinalis beispielsweise nach dem Verfahren von Walsmann und Markwardt (Thromb. Res. 40, 563, (1985) extrahierbare Polypeptid Hirudin oder ein nach einer rekombinanten Technologie, beispielweise nach dem Verfahren von J. Dodt et al. (FEBS Letters 202, 373, (1986) aus Microorganismen gewinnbares r-Hirudin oder r-Desulfatohirudin verwenden.

Das erfindungsgemässe Verfahren kann beispielsweise bei den nachstehend genannten Bestimmungen oder Untersuchungen verwendet werden:

Bestimmung der Aggregation, Sedimentation und Deformierbarkeit der Erythrozyten.

Untersuchung der Adhäsivität und der cytochemischen und immunologischen Eigenschaften der Leukozyten.

Untersuchung der Adhäsions-, Aggregations- und Freisetzungsreaktionen der Thrombozyten.

Zur Entnahme und Aufnahme der für die Bestimmung der Funktionen und Zustandsänderungen von Blutzellen und anderen Blutkorpuskeln zu verwendenden Blutproben werden Geräte oder Behälter verwendet, welche mit dem Inhibitor behandelt oder beschickt sind. Als Beispiele solcher Geräte und Behälter können Röhrchen, Spritzen, Kapillarröhrchen, Ampullen, Pipetten, Objektträger und Mikrotiterplatten aus Glas, Kunststoff oder Metall genannt werden.

Die vorliegende Erfindung betrifft somit auch Geräte und/oder Behälter, welche zur Entnahme und/oder Aufnahme von zur Bestimmung der Funktionen und Zustandsänderungen von Blutzellen und anderen Blutkorpuskeln zu verwendenden Blutproben bestimmt sind und welche dadurch gekennzeichnet sind, dass sie mit dem Inhibitor behandelt oder beschickt sind.

Die Beschickung mit Inhibitor kann dadurch erfolgen, dass man den zweckmässigerweise mit einer inerten Trägersubstanz vermischte, lyophilisierte oder getrocknete Produkt pulverförmig, nach Gewicht oder Volumen dosiert, in das Gerät oder den Behälter aufträgt oder einbringt oder indem man den Inhibitor als Lösung einfüllt, aufsprüht oder einsprüht und anschliessend trocknet.

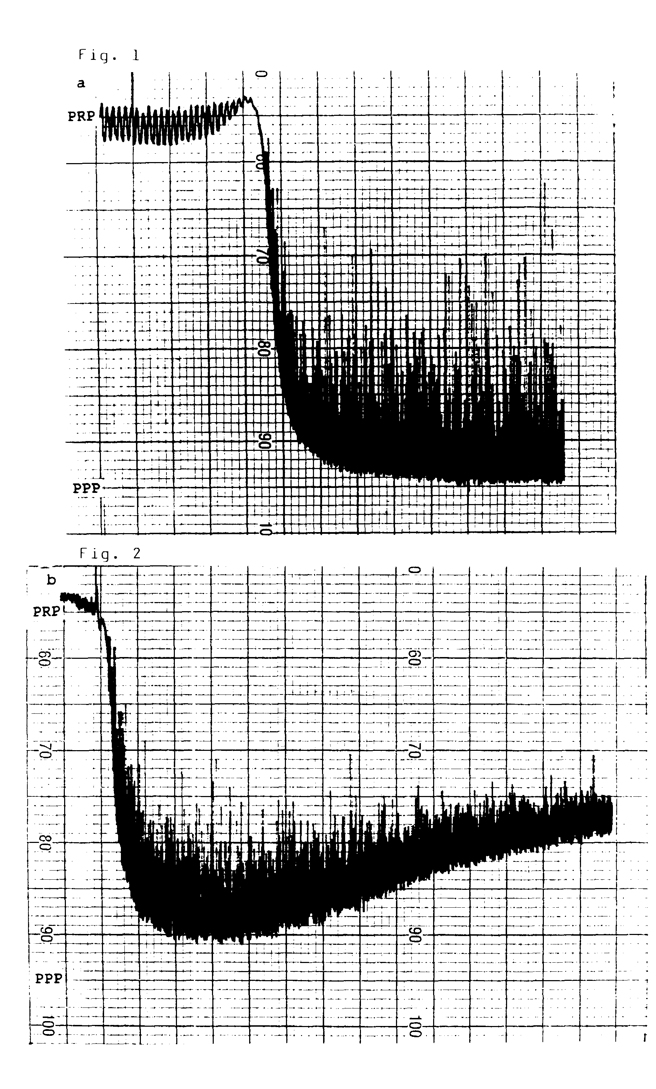

Die beiliegenden Zeichnungen 1 bis 3 sind Diagramme, welche die photometrisch registrierten Veränderungen der Lichtabsorption von plättchenreichem menschlichem Plasma während der durch drei verschiedene Stimulatoren ausgelösten Thrombozytenaggregation wiedergeben. In den Diagrammen gemäss Fig. 1 bis 3 betrifft die X-Achse den zeitlichen Ablauf und die Y-Achse die Lichtabsorptionsänderungen.

### Beispiel 1

Blutaufnahmeröhrchen aus Kunststoff mit einem Fassungsvermögen von 3 ml wurden bei einem Volumen von 2 ml mit einer Eichmarke versehen. In jedes Röhrchen wurde 0,1 ml einer wässrigen Lösung von 6000 Antithrombineinheiten (ATU) r-Hirudin pro ml einpipettiert, worauf die Röhrchen im Vacuum, bei 50-60°C getrocknet wurden. Die derart vorpräparierten Röhrchen wurden zur Aufnahme von Proben zu je 2 ml Venenblut verwendet. Das in diesen vorpräparierten Röhrchen gesammelte Blut blieb während über 8 Stunden ungeronnen, also während einer zur Durchführung der Bestimmung der Blutsenkungsgeschwindigkeit genügenden Zeitspanne.

### Beispiel 2

Blutaufnahmeröhrchen aus Kunststoff mit einem Fassungsvermögen von 3 ml wurden bei einem Volumen von 2 ml mit einer Eichmarke versehen. In jedes Röhrchen wurde 0,1 ml einer wässrigen Lösung von NAPAP, 2 µMol per ml, einpipettiert, worauf die Röhrchen im Vacuum bei 50-60°C getrocknet wurden. In derart vorpräparierte Röhrchen aufgenommene Blutproben blieben während mehr als 8 Stunden ungeronnen.

### Beispiel 3

Blutaufnahmeröhrchen aus Kunststoff mit einem Fassungsvermögen von 3 ml wurden bei einem Volumen von 2 ml mit einer Eichmarke versehen. In jedes Röhrchen wurde 0,1 ml einer Lösung von 300 ATU r-Hirudin und 1 µMol NAPAP per ml einpipettiert, worauf die Röhrchen im Vacuum bei 50-60°C getrocknet wurden. In derart vorpräparierte Röhrchen aufgenommene Blutproben blieben während über 8 Stunden ungeronnen.

### Beispiel 4

### Micromethode zur Bestimmung der Blutsenkungsgeschwindigkeit

Zur Durchführung einer Micromethode zur Bestimmung der Blutsenkungsgeschwindigkeit wurden Glaskapillaren mit einer Länge von 150 mm und einem inneren Durchmesser von 1 mm bei einer vom unteren Ende abgemessenen Höhe von 100 mm mit einer Eichmarke versehen und mit einer wässrigen Lösung von 5 µMol NAPAP, 6000 ATU r-Hirudin und 50 µl Glycerin per ml vorpräpariert, indem die Antikoagulantienlösung bis zur Eichmarke aufgesogen und wieder auslaufen gelassen wurde. Die Kapillaren wurden dann im Vacuum bei 50-60°C getrocknet. Aus der Fingerbeere entnommenes, in die vorpräparierten Kapillaren aufgesogenes Blut blieb während über 8 Stunden ungeronnen. Die mit diesen Kapillaren an 3 Normalpersonen bestimmten Blutsenkungsgeschwindigkeitswerte betrugen 5/9, 3/7 und 9/20 mm pro 1 bzw. 2 Stunden.

### Beispiel 5

### Nitroblautetrazolium (NBT)-Reaktion

Nitroblautetrazolium ist ein blassgelber Farbstoff, der nach Aufnahme durch bestimmte neutrophile Zellen zu Formazan abgebaut wird. Formazan erscheint als blauschwarze Ausscheidung im Cytoplasma dieser neutrophilen Zellen. Normalerweise nehmen weniger als 10% der neutrophilen Zellen diesen Farbstoff auf. Ein überraschender Anstieg der Farbstoffaufnahme wurde bei Patienten mit bakteriellen Infektionen beobachtet, während bei Vorliegen von Virus- oder Pilzinfektionen eine niedrige oder normale Anzahl an NBT-positiven neutrophilen Leukozyten gefunden werden.

In Blutentnahmeröhrchen gemäss Beispiel 1 gesammeltes Venenblut wurde während 8 Minuten bei 1500 x g zentrifugiert. Die zwischen der Erythrozyten- und Plasmazone befindliche, leukozytenhaltige Zwischenschicht wurde mit einer Kunststoffpipette gesammelt und in einem Kunststoffreagenzglas mit Nitroblautetrazolium-Reagenz (0,2% NBT in Phosphatpuffer pH 7,2) während 30 Minuten bei 37°C inkubiert. Nach beendeter Reaktion wurden Zellproben auf Objektträger ausgestrichen, mittels Methanol fixiert, getrocknet und mit Safranin während 2 Minuten gegengefärbt. Die Ausstriche wurden unter dem Mikroskop bei 1000-facher Vergrösserung ausgewertet, indem man 100 neutrophile Zellen auf ihre Färbung durch das NBT-Reaktionsprodukt Formazan prüfte und daraus den Prozentsatz an NBT-positiven Zellen errechnete. Bei drei normalen Blutspendern wurden je 6,2%, 5,7% und 8,9% NBT-positive Zellen gefunden.

### Beispiel 6

### Messung der Plättchenaggregation

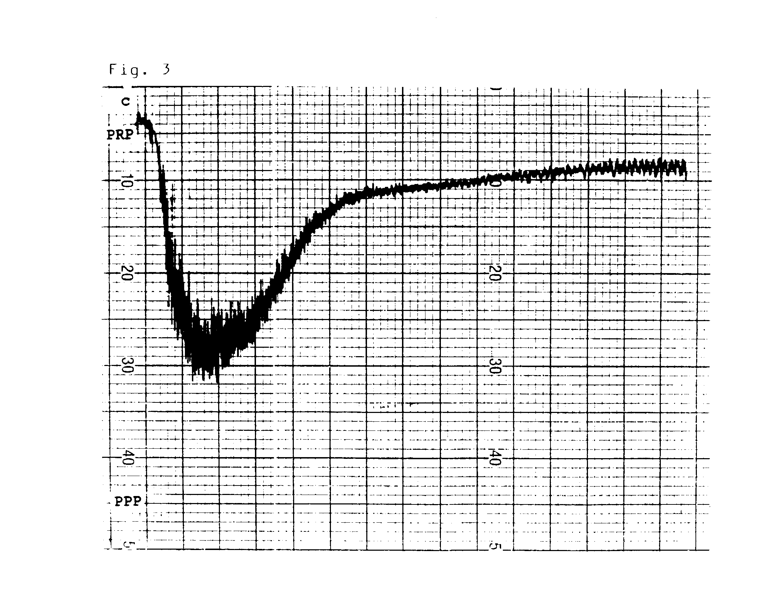

In Zentrifugenröhrchen aus Polypropylen mit einem Fassungsvermögen von 15 ml und einer Eichmarke bei einem Volumen von 10 ml wurden je 0,2 ml einer wässrigen Lösung von 30'000 ATU r-Hirudin pro ml pipettiert und lyophilisiert. In derart vorbehandelte Röhrchen wurde frisch entnommenes Venenblut bis zur Eichmarke eingefüllt. Die Röhrchen wurden mit Kunststoffolie verschlossen und fünfmal vorsichtig um 90° gekippt, um eine Durchmischung von Blut und Hirudin zu gewährleisten. Hierauf wurde während 8 Minuten bei 1000 x g zentrifugiert und das überstehende, plättchenreiche Plasma mit einer Kunststoffpipette abgehebert und in einem Kunststoffröhrchen gesammelt. An diesem plättchenreichen Hirudinplasma wurde photometrisch, in einer magnetisch gerührten Küvette, die Aggregation der Plättchen, welche eine Abnahme der Lichtabsorption bewirkt, nach Zusatz von Kollagen (a) (siehe Fig. 1), Thrombocytin (b) (siehe Fig. 2) und Adenosindiphosphorsäure (c) (siehe Fig. 3) gemessen.

### Beispiel 7

### Messung der Gerinnselretraktion

Je 0,6 ml plättchenreiches Hirudinplasma gemäss Beispiel 6 wurden in zuvor ausgeglühte und erkalten gelassene Reagenzgläser mit einem Durchmesser von 9,75 mm pipettiert, mit 0,2 ml einer Lösung von Adenosindiphosphat (ADP) in physiologischer Kochsalzlösung bezw. mit 0,2 ml physiologische Kochsalzlösung (Leerwert) und hierauf mit 0,1 ml wässriger Batroxobinlösung, welche 20 BU Batroxobin per ml enthielt, versetzt. Die Proben wurden während 2 Stunden ohne zu bewegen in einem Wasserbad bei 37°C gehalten, worauf das ausgepresste Serum vom retrahierten Gerinnsel abgetrennt und dessen Volumen mittels einer µl-Spritze gemessen wurde. Aus dem ursprünglichen Gerinnselvolumen und dem gemessenen Serumvolumen wurde die Gerinnselretraktion in Prozent errechnet. Sie betrug für den Kontrollversuch 0 und für den Versuch mit ADP 94%.

### Beispiel 8

### Bestimmung der Blutsenkungsgeschwindigkeit bei Antikoagulantien-Überdosis

Blutentnahmeröhrchen aus Kunststoff wurden gemäss Beispiel 1 bis 3 mit Hirudin (H), NAPAP (N) bezw. Hirudin und NAPAP (HN) vorpräpariert und bei 2 ml und bei 1,5 ml Volumen mit je einer Eichmarke versehen. Zum Vergleich wurden käufliche, mit Citratlösung (C) beschickte, mit einer 2 ml-Eichmarke versehene Blutentnahmeröhrchen mit einer zusätzlichen Marke bei einem Volumen von 1,5 ml versehen. Mit Bezug auf das Antikoagulans wurden also vier verschiedene Röhrchentypen (H, N, HN und C) erhalten. Von freiwilligen Spendern wurden nun jeweils acht Blutproben in die vier Röhrchentypen entnommen. Jeder Röhrchentyp wurde mit Venenblut je eines Probanden einmal auf das Sollvolumen von 2 ml und einmal auf das Mindervolumen von 1,5 ml aufgefüllt. Die mit einem zu geringen Blutvolumen aufgefüllten Citratröhrchen lieferten gegenüber den mit dem Sollvolumen beschickten Citratröhrchen viel zu geringe BSG-Werte, während an allen übrigen, mit den Röhrchentypen H, N und HN gesammelten Blutproben, vom Sammelvolumen unabhängig, bei jedem Probanden nicht signifikant voneinander abweichende BSG-Werte bestimmt wurden.

**Tabelle I**

| Volumen | Blutsenkungsgeschwindigkeit (mm/Std.) | | | |
|---|---|---|---|---|
| | H | N | HN | C |
| 2 ml | 8 ± 6,7 | 8 ± 6,2 | 7 ± 6,2 | 7 ± 6,5 |
| 1,5 ml | 8 ± 6,8 | 7 ± 6,4 | 8 ± 6,4 | 5 ± 4,3 |

## Patentansprüche

1. Verfahren zur Bestimmung von meizothrombin- und thrombinunabhängigen Funktionen und Zustandsänderungen von Blutzellen und Blutkorpuskeln, dadurch gekennzeichnet, dass eine Blutprobe mit einem zweiwertige Kationen nicht bindenden Meizothrombininhibitor und Thrombininhibitor behandelt und die meizothrombin- und thombinunabhängigen Funktionen und Zustandsänderungen von Blutzellen in der Blutprobe gemessen werden.

2. Verfahren nach Patentanspruch 1, dadurch gekennzeichnet, dass der Inhibitor Hirudin oder Desulfatohirudin ist.

3. Verfahren nach Patentanspruch 1, dadurch gekennzeichnet, dass der Inhibitor eine Verbindung der Formel 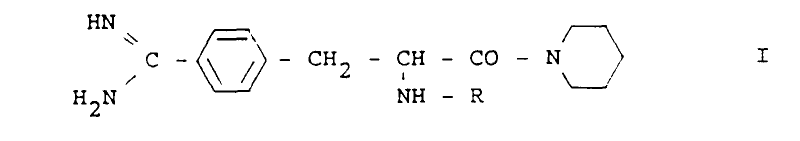 in welcher R eine Toluolsulfonylglycyl-, α-Naphthylsulfonylglycyl- oder β-Naphtylsulfonylglycyl-Gruppe darstellt, ist.

4. Verfahren nach Patentansprüchen 1- 3, dadurch gekennzeichnet, dass der Inhibitor ein Gemisch von Hirudin oder Desulfatohirudin mit einer Verbindung der Formel I ist.

5. Anwendung des Verfahrens gemäss den Patentansprüchen 1 - 4 bei der Bestimmung der Aggregation, Sedimentation und Deformierbarkeit der Erythrozyten.

6. Anwendung des Verfahrens gemäss Patentansprüchen 1 - 4 bei der Untersuchung der Adhäsivität und der cytochemischen und immunologischen Eigenschaften der Leukozyten.

7. Anwendung des Verfahrens gemäss Patentansprüchen 1 - 4 bei der Untersuchung der Aggregations- und Freisetzungsreaktionen der Thrombozyten.

## Claims

1. Method for the determination of meizothrombin- and thrombin-independent functions and state changes of blood cells and blood corpuscles which comprises treating a blood sample with a meizothrombin inhibitor and thrombin inhibitor that does not bind bivalent cations and measuring the meizothrombin- and thrombin-independent functions and state changes of blood cells in the blood sample.

2. Method according to claim 1, wherein the inhibitor is hirudin or desulfatohirudin.

3. Method according to claim 1, wherein the inhibitor is a compound of formula 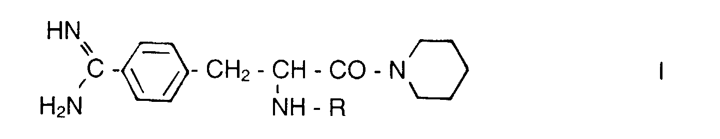 where R represents a toluenesulfonylglycyl, an α-naphthylsulfonylglycyl or a β-naphthylsulfonylglycyl group.

4. Method according to claims 1 to 3, wherein the inhibitor is a mixture of hirudin or desulfatohirudin with a compound of formula 1.

5. Use of the method according to claims 1 to 4 for the determination of aggregation, sedimentation, and deformability of erythrocytes.

6. Use of the method according to claims 1 to 4 for the investigation of the adhesivity and of the cytochemical and immunological properties of leukocytes.

7. Use of the method according to claims 1 to 4 for the investigation of aggregation and release reactions of thrombocytes.

## Revendications

1. Procédé de détermination de fonctions et modifications d'état des cellules et globules sanguins indépendantes de meizothrombine et de thrombine, caractérisé en ce que l'on traite un échantillon de sang avec un inhibiteur de meizothrombine et de thrombine qui ne lie pas les cations bivalents et que l'on mesure les fonctions et modifications d'état des cellules sanguines indépendantes de meizothrombine et de thrombine dans l'échantillon de sang.

2. Procédé suivant la revendication 1, caractérisé en ce que l'inhibiteur est de l'hirudine ou de la désulfatohirudine.

3. Procédé suivant la revendication 1, caractérisé en ce que l'inhibiteur est un composé de formule 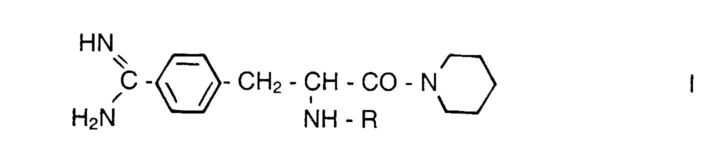 où R représente un groupe toluènesulfonylglycyle, α-naphtylsulfonylglycyle ou β-naphtylsulfonylglycyle.

4. Procédé suivant les revendications 1 à 3, caractérisé en ce que l'inhibiteur est un mélange d'hirudine ou de désulfatohirudine avec un composé de formule I.

5. Application du procédé suivant les revendications 1 à 4 pour la détermination de l'agrégation, de la sédimentation et de la déformabilité des érythrocytes.

6. Application du procédé suivant les revendications 1 à 4 pour l'étude de l'adhésivité et des propriétés cytochimiques et immunologiques des leucocytes.

7. Application du procédé suivant les revendications 1 à 4 pour l'étude des réactions d'agrégation et de libération des thrombocytes.
